# EUROPÄISCHE PATENTSCHRIFT

(11) **EP 0 943 304 B1**
(45) Veröffentlichungstag und Bekanntmachung des Hinweises auf die Patenterteilung: **02.03.2005**
(21) Anmeldenummer: 98250441.7
(22) Anmeldetag: 17.12.1998
(51) Int. Cl.: A61F 2/34, B23G 5/06

(54) **Schraubpfanne für eine Hüftgelenksprothese**
Threaded acetabular cup for a hip joint prosthesis
Coque acétabulaire filetée pour une prothèse de l'articulation de la hanche

(30) Priorität: 17.03.1998 DE 19812874
(43) Veröffentlichungstag der Anmeldung: 22.09.1999
(73) Patentinhaber: Biomet Merck Deutschland GmbH, 14167 Berlin (DE)
(72) Erfinder: Schütz, Rüdiger, Dr. med., 30539 Hannover (DE); Kranz, Curt, Dr.-Ing., 14532 Stahnsdorf (DE); Calisse, Jorge, Dr.-Ing., 14169 Berlin (DE)
(74) Vertreter: Gross, Felix, Dr.

(56) Entgegenhaltungen:
- EP-A- 0 480 551
- EP-A- 0 482 320
- WO-A-95/18586
- DE-C- 19 514 455
- FR-A- 2 610 512
- FR-A- 2 639 822

## Beschreibung

Die Erfindung betrifft eine Schraubpfanne für eine Hüftgelenksprothese gemäß dem Oberbegriff des Anspruchs 1 sowie ein Verfahren zu deren Herstellung.

Derartige Schraubpfannen werden bei der Hüftgelenksoperation in eine zuvor gefräste Aussparung im Hüftknochen des Patienten eingesetzt, die der Mantelfläche des Grundkörpers der Schraubpfanne entspricht. Anschließend wird die Schraubpfanne zur zementlosen Fixierung in den Hüftknochen eingeschraubt, bis der Grundkörper in der Aussparung am Hüftknochen anliegt und so ein weiteres Einschrauben verhindert. Das Gewinde ist dabei häufig selbstschneidend ausgeführt, so daß sich eine weitere Vorbearbeitung des Hüftknochens erübrigt. Um möglichst viel von der Knochensubstanz des Hüftknochens zu erhalten und so eine zu große Schwächung des Hüftknochens zu vermeiden, weist das Gewinde in der Regel im Axialschnitt der Schraubpfanne in relativ großem axialem Abstand zueinander angeordnete schlanke Gewindezähne auf. Der Gewindegrund zwischen den Gewindezähnen ist dabei der Mantelfläche des Grundkörpers mehr oder weniger genau nachgebildet und liegt im Idealfall in der Aussparung flächig am Hüftknochen an.

Bei den bekannten Schraubpfannen mit konischem Grundkörper kann der Gewindegrund zwar problemlos der konischen Mantelfläche des Grundkörpers angepaßt werden, so daß der Gewindegrund im Idealfall in einer mit einem entsprechenden konischen Fräser erzeugten Aussparung vollflächig am Hüftknochen anliegt. Bei diesen Schraubpfannen besteht jedoch der Nachteil, daß beim Einschrauben der Schraubpfanne schon kleinste Winkelabweichungen zwischen der Längsachse der Ausnehmung und der Längsachse der Schraubpfanne dazu führen, daß der Grundkörper der Schraubpfanne nicht mehr vollflächig am Hüftknochen anliegt. Die Gelenklast wird dann nur noch über die Gewindeflanken und mehr oder weniger kleine Bereiche des Grundkörpers in den Hüftknochen eingeleitet, wodurch es zu einer unerwünschten lokalen Überlastung des Hüftknochens und damit zu einer Beschädigung des Knochens sowie zum Lockern der Pfanne kommen kann.

Bei den bekannten Schraubpfannen mit halbkugelförmigem Grundkörper sind die genannten Winkelabweichungen beim Einschrauben der Schraubpfanne in den Hüftknochen unkritisch, da es aufgrund der einander entsprechenden sphärischen Konturen des Grundkörpers der Schraubpfanne und der Ausnehmung im Hüftknochen zu keinem Verkanten wie bei den konischen Schraubpfannen kommen kann. Die bekannten sphärischen Schraubpfannen weisen jedoch den Nachteil auf, daß die Anpassung des Gewindegrundes an die sphärische Kontur des Grundkörpers nur unvollständig und mit sehr aufwendigen Mitteln möglich ist.

So offenbart beispielsweise die europäische Patentanmeldung 0 480 551 A1 eine gattungsgemäße, sphärische Schraubpfanne, bei der die sphärische Kontur des Grundkörpers im Gewindegrund durch eine Anzahl im Axialschnitt zueinander geneigt verlaufender Teilflächen polygonzugartig angenähert ist. Die Teilflächen bilden dabei an ihrem Stoß jeweils eine stumpfwinklige Dachkante. Im Gewindegrund liegt diese Schraubpfanne somit ebenfalls nicht vollflächig sondern nur im Bereich der Dachkanten am Hüftknochen an. Um in der sphärischen Ausnehmung ein möglichst großflächiges Anliegen der Schraubpfanne am Hüftknochen zu erzielen, ist es bei dieser Schraubpfanne erforderlich, die sphärische Kontur des Grundkörpers durch viele zueinander geneigte Teilflächen möglichst genau anzunähern, womit allerdings ein erheblich erhöhter Fertigungsaufwand erforderlich ist.

Ein weiterer Nachteil der bekannten gattungsgemäßen Schraubpfannen besteht darin, daß schon relativ geringe Abweichungen des Grundkörpers der Schraubpfanne oder der Aussparung im Hüftknochen von der Idealgeometrie dazu führen können, daß unter Umständen große Bereiche des Grundkörpers in der Aussparung nicht am Hüftknochen anliegen und somit nicht an der Lasteinleitung in den Hüftknochen teilnehmen. Gerade beim Ausfräsen der Aussparung im Hüftknochen, das der Chirurg in der Regel von Hand vornimmt, kann es selbst bei erhöhter Sorgfalt, z. B. durch nicht exakt axiale Kraftausübung auf den Knochenfräser oder durch unbeabsichtigtes Schwenken der Fräserachse während des Fräsens, schnell zu derartigen Abweichungen der Aussparung von der Idealgeometrie kommen.

Dokument FR-A-2 610 512 offenbart eine Schraubpfanne gemäß des Oberbegrifß von Anspruch 1.

Der Erfindung liegt deshalb die Aufgabe zugrunde, eine gattungsgemäße Schraubpfanne zur Verfügung zu stellen, die die genannten Nachteile nicht oder zumindest in geringerem Maße aufweist, wobei sie insbesondere bei einfacher Herstellbarkeit der Schraubpfanne eine gleichmäßige Einleitung der Gelenklasten in den Hüftknochen und eine zuverlässige Fixierung sicherstellt. Weiterhin liegt der Erfindung die Aufgabe zugrunde, ein Herstellungsverfahren für eine derartige Schraubpfanne anzugeben.

Die Aufgabe wird, ausgehend von einer Schraubpfanne gemäß dem Oberbegriff des Anspruchs 1, durch die im kennzeichnenden Teil des Anspruchs 1 angegebenen Merkmale gelöst.

Die Erfindung schließt die technische Lehre ein, daß man eine schnell und einfach herzustellende Schraubpfanne mit guter Krafteinleitung in den Hüftknochen erhält, wenn auf der Außenseite des Grundkörpers der Schraubpfanne zwischen zwei benachbarten Flanken des ersten Gewindes wenigstens ein den Gewindegrund des ersten Gewindes ausfüllendes zweites Gewinde vorgesehen ist, dessen lokale Flankenhöhe senkrecht zur Mantelfläche des Grundkörpers über im wesentlichen die gesamte Länge des zweiten Gewindes wesentlich geringer ist als die lokale Flankenhöhe der beiden benachbarten Flanken des ersten Gewindes. Unter lokaler Flankenhöhe soll hierbei der in dem jeweiligen Punkt des Gewindes senkrecht zur Mantelfläche des Grundkörpers gemessene Abstand der Gewindespitze zur Mantelfläche des Grundkörpers verstanden werden. Weisen die beiden benachbarten Flanken des ersten Gewindes unterschiedliche Flankenhöhe auf, so ist die lokale Flankenhöhe des zweiten Gewindes geringer als die lokale Flankenhöhe der kürzeren der beiden benachbarten Flanken. Der Betrag, um den die lokale Flankenhöhe des zweiten Gewindes geringer ist als die lokale Flankenhöhe der beiden benachbarten Flanken des ersten Gewindes, ist dabei ausreichend groß gewählt, so daß eine wesentliche Schwächung des Hüftknochens durch das zweite Gewinde vermieden ist. Die Steigung des zweiten Gewindes entspricht dabei aus Gründen der einfacheren Fertigung vorzugsweise der Steigung des ersten Gewindes.

Das im Gewindegrund des ersten Gewindes angeordnete zweite Gewinde stellt gegenüber den bekannten Schraubpfannen eine vergrößerte Kontaktfläche und besseren Kontakt zwischen der Schraubpfanne und dem Hüftknochen sicher. Je nach Flankenhöhe des zweiten Gewindes ergibt sich der verbesserte Kontakt zwischen Schraubpfanne und Hüftknochen aus zwei sich gegebenenfalls überlagernden Effekten des zweiten Gewindes.

Weist das zweite Gewinde zum einen eine so geringe Flankenhöhe auf, daß es nicht in den Hüftknochen eingeschraubt wird, sondern die Spitzen des zweiten Gewindes nur mehr oder weniger stark gegen den Hüftknochen gedrückt werden, so regt diese mit Belastung des Gelenkes über die Spitzen des zweiten Gewindes übertragene, schwellende Drucklast das Knochengewebe zu verstärktem Wachstum an. Hierbei ist es für das Wachstum förderlich, daß die Drucklast nicht großflächig sondern nur über die relativ schmalen Gewindespitzen des zweiten Gewindes in den Hüftknochen eingeleitet wird. Das somit in die Gewindegänge des zweiten Gewindes einwachsende Knochengewebe verbessert dabei den Kontakt zwischen Schraubpfanne und Hüftknochen erheblich gegenüber den bekannten Schraubpfannen mit mehr oder weniger glattem Gewindegrund zwischen den Flanken des ersten Gewindes. Dies gilt auch dann, wenn die Schraubpfanne nicht exakt in Axialrichtung der Aussparung im Hüftknochen eingeschraubt wird oder die Aussparung im Hüftknochen in der oben beschriebenen Weise von der Idealgeometrie abweicht und der Gewindegrund des ersten Gewindes somit, wie eingangs geschildert, nicht vollständig in der Aussparung am Hüftknochen anliegt. Aufgrund der naturgemäß relativ spitzwinkligen Ausführung werden die Gewindespitzen des zweiten Gewindes beim Einschrauben der Schraubpfanne ohne weiteres in den Hüftknochen gedrückt bis der Gewindegrund in ihrem Bereich am Hüftknochen anliegt und ein weiteres Einschrauben behindert. In Bereichen, in denen Zwischenräume zwischen Hüftknochen und Gewindegrund auftreten, überbrückt das zweite Gewinde derartige Zwischenräume. Der oben geschilderte Effekt tritt auch hier ein, da schon relativ leichter Kontakt der Gewindespitzen mit dem Hüftknochen ausreicht, um das Knochenwachstum anzuregen und somit den Zwischenraum zwischen Hüftknochen und Gewindegrund auszufüllen. Mehr oder weniger unvermeidbare Fehler beim Einsetzen der Schraubpfanne bzw. beim Ausfräsen der Aussparung im Hüftknochen aber auch Abweichungen des Grundkörpers der Schraubpfanne von der Idealgeometrie werden hierdurch in einfacher Weise zuverlässig kompensiert.

Weist das im Gewindegrund des ersten Gewindes angeordnete zweite Gewinde zum anderen eine so große Flankenhöhe auf, daß es ebenfalls in den Hüftknochen eingeschraubt wird, so stellt es durch seine in den Knochen eingreifenden Flanken gegenüber den bekannten Schraubpfannen eine vergrößerte Kontaktfläche zwischen der Schraubpfanne und dem Hüftknochen sicher. Dies gilt insbesondere gerade dann, wenn die Schraubpfanne nicht exakt in Axialrichtung der Aussparung im Hüftknochen eingeschraubt wird oder die Aussparung im Hüftknochen in der oben beschriebenen Weise von der Idealgeometrie abweicht und der Gewindegrund des ersten Gewindes somit, wie eingangs geschildert, nicht vollständig in der Aussparung am Hüftknochen anliegt. Das zweite Gewinde überbrückt derartige eventuelle Zwischenräume im Gewindegrund des ersten Gewindes zwischen der Schraubpfanne und dem Hüftknochen. Der in den Hüftknochen eingreifende Anteil der Gewindeflanken des zweiten Gewindes stellt dann immer noch eine ausreichend große Fläche zur schonenden Einleitung der Gelenklasten in den Hüftknochen sicher. Mehr oder weniger unvermeidbare Fehler beim Einsetzen der Schraubpfanne bzw. beim Ausfräsen der Aussparung im Hüftknochen aber auch Abweichungen des Grundkörpers der Schraubpfanne von der Idealgeometrie werden hierdurch in einfacher Weise zuverlässig kompensiert.

Es versteht sich, daß sich bei entsprechender Flankenhöhe des zweiten Gewindes und mehr oder weniger starker Ausprägung der genannten Abweichungen beide genannten Effekte auch einander überlagert auftreten können.

Da die Herstellung des zweiten Gewindes insbesondere bei Schraubpfannen mit sphärischem Grundkörper bedeutend einfacher zu bewerkstelligen ist als die oben beschriebene aufwendige Anpassung des Gewindegrundes des ersten Gewindes an den Grundkörper, reduziert sich auch der Herstellungsaufwand für die erfindungsgemäßen Schraubpfannen gegenüber den bekannten Schraubpfannen zum Teil erheblich.

Die Flankenhöhe des zweiten Gewindes ist dabei so gering gewählt, daß sichergestellt ist, daß der Hüftknochen nicht wesentlich durch das zweite Gewinde geschwächt wird, wie dies beispielsweise der Fall wäre, wenn die Flankenhöhe des zweiten Gewindes etwa im Bereich der Flankenhöhe des ersten Gewindes läge. In diesem Fall würde zum einen unvorteilhafterweise eine große Menge lebenden Hüftknochengewebes entfernt, zum anderen wäre die Festigkeit des Hüftknochens durch den aufgrund des relativ geringen Abstandes zwischen benachbarten Gewindespitzen verringerten Spannungsquerschnitt und die erhöhte Kerbwirkung im Grund des in den Hüftknochen geschnittenen Gegengewindes erheblich reduziert. Die erfindungsgemäß ausreichend geringe Flankenhöhe des zweiten Gewindes bewirkt, daß möglichst viel lebendes Knochengewebe erhalten bleibt und somit die mechanischen Eigenschaften des Knochens im Einschraubbereich so weit wie möglich erhalten bleiben.

Die fakultativen Merkmale günstiger Weiterbildungen der Erfindung sind in den Unteransprüchen angegeben, welche den Gegenstand der erfindungsgemäßen Lösung nicht einschränken.

So ist die lokale Flankenhöhe des zweiten Gewindes ist vorzugsweise so gewählt, daß sie um wenigstens ein Drittel, vorzugsweise wenigstens die Hälfte, geringer ist als die lokale Flankenhöhe der gegebenenfalls kürzeren der beiden benachbarten Flanken des ersten Gewindes. Durch diese Abmessungen ist eine wesentliche Schwächung des Hüftknochens durch das zweite Gewinde zuverlässig ausgeschlossen. Um eventuelle Lücken zwischen der Schraubpfanne und dem Hüftknochen zuverlässig überbrücken zu können ist die lokale Flankenhöhe des zweiten Gewindes vorzugsweise im wesentlichen um höchstens 95% geringer gewählt als die lokale Flankenhöhe der gegebenenfalls kürzeren der beiden benachbarten Flanken des ersten Gewindes. Dabei versteht es sich, daß das zweite Gewinde zum einen auch über seine Länge eine variierende Flankenhöhe aufweisen kann. Zum anderen können auch benachbarte Gänge des zweiten Gewindes unterschiedliche Flankenhöhe aufweisen, insbesondere können die durch die Flanken des ersten Gewindes getrennten Gänge des zweiten Gewindes unterschiedliche Flankenhöhe aufweisen.

Bei den erfindungsgemäßen Schraubpfannen kann es sich um prinzipiell in herkömmlicher Weise aufgebaute Schraubpfannen aus einem biokompatiblen Material, insbesondere biokompatiblem Metall oder einer entsprechenden Metallegierung, handeln, die im Pfanneninneren eine Aussparung zur Aufnahme eines Inlays, beispielsweise aus Kunststoff etc., aufweisen, das dann die Gleitfläche für den im Femur verankerten Kugelkopf des künstlichen Hüftgelenkes bildet.

Bei vorteilhaften Ausführungen der erfindungsgemäßen Schraubpfanne ist das erste, alternativ oder vorzugsweise zusätzlich auch das zweite Gewinde selbstschneidend ausgebildet. Gegenüber Gewinden, deren Gegengewinde zuvor mit entsprechenden Werkzeugen in den Hüftknochen eingeschnitten werden muß, ergibt sich dabei der Vorteil, daß über das gesamte Gewinde eine gute flächige Paarung zwischen dem jeweiligen selbstschneidenden Gewinde und dem Hüftknochen vorliegt, die nicht durch Formabweichungen zwischen dem Schneidwerkzeug und der Schraubpfanne oder durch Fehler beim Gewindeschneiden beeinflußt wird. Es versteht sich jedoch, daß sich bei entsprechend geringer Flankenhöhe eine selbstschneidende Ausführung des zweiten Gewindes erübrigt.

Bei günstigen Varianten der erfindungsgemäßen Schraubpfanne ist das erste Gewinde, alternativ oder zusätzlich auch das zweite Gewinde mehrgängig ausgebildet, wobei vorzugsweise die Gangzahl des zweiten Gewindes einem ganzzahligen Vielfachen der Gangzahl des ersten Gewindes entspricht. Hierbei soll die angegebene Gangzahl des zweiten Gewindes als auf ein einziges zweites Gewinde bezogen verstanden werden, das im Grund des ersten Gewindes angeordnet ist und dessen Gänge auf die einzelnen Gänge des ersten Gewindes verteilt angeordnet sind. Weiter vorzugsweise ist das erste Gewinde in bekannter Weise zweigängig und das zweite Gewinde wenigstens viergängig ausgebildet ist. Im Sinne der oben genannten Terminologie sind also zwischen zwei Flanken des ersten Gewindes jeweils wenigstens zwei Flanken des zweiten Gewindes angeordnet. Hierdurch wird eine gute und gleichmäßige Ausfüllung des Gewindegrundes des ersten Gewindes mit einem zweiten Gewinde ausreichend geringer Flankenhöhe erzielt.

Vorteilhafte Weiterbildungen der Schraubpfanne zeichnen sich dadurch aus, daß das zweite Gewinde derart ausgebildet und angeordnet ist, daß der Zwischenraum im Gewindegrund zwischen zwei Flanken des ersten Gewindes im Axialschnitt der Schraubpfanne durch den zwischen den zwei Flanken des ersten Gewindes angeordneten Gewindegang des zweiten Gewindes oder die zwischen den zwei Flanken des ersten Gewindes angeordneten Gewindegänge des zweiten Gewindes im wesentlichen gleichmäßig unterteilt ist. Hierdurch wird zum einen eine besonders gleichmäßige Einleitung der Gelenklasten in den Hüftknochen erreicht. Zum anderen lassen sich derart gleichmäßige Teilungen besonders einfach mit nur wenigen Werkzeugen herstellen.

Bei besonders günstigen Varianten ist der Übergangsbereich zwischen zwei Flanken im Gewindegrund im Axialschnitt der Schraubpfanne im wesentlichen bogenförmig ausgebildet, beispielsweise ellipsen- oder kreisbogenförmig, wodurch die Kerbwirkung im Fußbereich der Gewindezähne verringert ist. Bei herstellungstechnisch besonders bevorzugten Varianten ist der Übergangsbereich im wesentlichen kreisbogenförmig ausgebildet, wobei er jeweils im wesentlichen tangential in eine der beiden Flanken mündet und einen Krümmungsradius aufweist, der höchstens im wesentlichen der halben Flankenhöhe des zweiten Gewindes entspricht. Dank dieses einfachen kreisbogenförmigen Übergangsbereiches gestaltet sich zum einen auch der diesen Bereich des ersten bzw. zweiten Gewindes erzeugende Abschnitt des bzw. der Bearbeitungswerkzeuge ebenfalls besonders einfach. So weist beispielsweise der diesen Bereich des ersten bzw. zweiten Gewindes erzeugende Werkzeugabschnitt bei einem Drehmeißel eine einfache ebenfalls kreisbogenförmige Schneidkante auf. Zum anderen ist eine Anpassung des Gewindegrundes an die Kontur des Grundkörpers besonders einfach zu bewerkstelligen, da der den Gewindegrund bearbeitende kreisbogenförmige Werkzeugabschnitt problemlos über den gesamten Gewindeverlauf tangential zur Kontur des Grundkörpers geführt werden kann, so daß der Gewindegrund der Kontur des Grundkörpers folgt. Der begrenzte Ausrundungsradius dient dabei zur Sicherstellung einer ausreichenden Länge des jeweiligen geraden Flankenabschnittes, der den Flankenwinkel des zweiten Gewindes und damit die Trag- und gegebenenfalls auch die Schneideigenschaften des zweiten Gewindes bestimmt.

Bei bevorzugten Ausführungen der erfindungsgemäßen Schraubpfanne verlaufen die distalen Flanken des ersten Gewindes und die distalen Flanken des zweiten Gewindes im Axialschnitt der Schraubpfanne zur Achse der Schraubpfanne um einen Winkel von im wesentlichen 70° bis 90°, vorzugsweise 75° bis 85°, geneigt. Die proximalen Flanken des ersten Gewindes und die proximalen Flanken des zweiten Gewindes verlaufen im Axialschnitt der Schraubpfanne ebenfalls um einen Winkel von im wesentlichen 70° bis 90°, vorzugsweise 75° bis 85°, zur Achse der Schraubpfanne geneigt. Hierdurch ergibt sich sowohl hinsichtlich der Festigkeit der Gewinde als auch hinsichtlich der Werkstoffpaarung aus Schraubpfanne und Knochen ein besonders günstiger Schlankheitsgrad der Gewindeflanken.

Vorzugsweise verlaufen dabei die distalen und proximalen Flanken des ersten Gewindes sowie die distalen und proximalen Flanken des zweiten Gewindes im Axialschnitt der Schraubpfanne zur Achse der Schraubpfanne um betragsmäßig im wesentlichen denselben Winkel geneigt. Aufgrund dieser Symmetrie ergibt sich eine besonders einfache Gestaltung und Führung des bzw. der die Gewinde erzeugenden Werkzeuge, mithin also eine besonders kostengünstige Herstellung der Schraubpfannen. Dieser Vorteil hinsichtlich der Gestaltung und Führung des bzw. der die Gewinde erzeugenden Werkzeuge erhöht sich noch weiter, wenn vorzugsweise die distalen Flanken des ersten Gewindes und die distalen Flanken des zweiten Gewindes sowie die proximalen Flanken des ersten Gewindes und die proximalen Flanken des zweiten Gewindes zueinander im wesentlichen parallel verlaufen. Zur Herstellung des ersten und zweiten Gewindes ist es dann möglich, ein und dieselbe Werkzeuggeometrie, insbesondere dasselbe bzw. dieselben Werkzeuge zu verwenden.

Besonders einfach hinsichtlich der Fertigung aber auch besonders günstig hinsichtlich der Einleitung der Gelenklasten in den Hüftknochen gestalten sich Ausführungen der Schraubpfanne, bei denen die Spitzen des ersten Gewindes über im wesentlichen die gesamte Länge des ersten Gewindes auf einer zur Mantelfläche des Grundkörpers im wesentlichen parallelen ersten Fläche liegen und die Spitzen des zweiten Gewindes über im wesentlichen die gesamte Länge des zweiten Gewindes auf einer zur Mantelfläche des Grundkörpers im wesentlichen parallelen zweiten Fläche liegen. Die Flanken des ersten bzw. des zweiten Gewindes weisen dabei dann über die Länge des ersten bzw. des zweiten Gewindes jeweils dieselbe Flankenhöhe auf. Hierbei sind insbesondere die genannten vorteilhaften Varianten mit konstanten Neigungswinkeln der Flanken des ersten und zweiten Gewindes zur Achse der Schraubpfanne besonders einfach zu fertigen, da das bzw. die Werkzeuge zur Erzeugung des ersten bzw. zweiten Gewindes durch Parallelverschiebung auf einfachen, parallel zur Mantelfläche des Grundkörpers verlaufenden Bahnkurven geführt werden müssen.

Die im wesentlichen zur Pfannenachse rotationssymmetrische Mantelfläche des Grundkörpers kann im wesentlichen die Form eines Kegelstumpfes, eines Ellipsoidteiles oder eines Paraboloidteiles aufweisen. Besonders vorteilhafte Varianten der Schraubpfanne zeichnen sich durch eine im wesentlichen halbkugelförmige Mantelfläche des Grundkörpers aus. Diese kugeligen Schraubpfannen nähern die Gelenkgeometrie des gesunden Hüftgelenks besser an als konische Varianten. Sie erfordern daher zum einen vorteilhafterweise die Resektion einer geringeren Menge lebenden Knochengewebes und weisen eine dem gesunden Gelenk ähnlichere Lasteinleitung in den Hüftknochen auf.

Die Erfindung betrifft weiterhin ein Verfahren zur spanenden Herstellung einer erfindungsgemäßen Schraubpfanne, bei dem die Schraubpfanne in einem ersten Teilschritt zur Erzeugung des ersten Gewindes mit wenigstens einem ersten Werkzeug und einem zweiten Werkzeug bearbeitet wird. Die restlichen Flanken des zweiten Gewindes können dabei im wesentlichen zeitgleich während des ersten Teilschrittes hergestellt werden. Alternativ kann ihre Herstellung auch in einem zweiten Teilschritt erfolgen. Das erste Werkzeug ist dabei vorzugsweise derart ausgebildet, daß es gleichzeitig die distale Flanke des ersten Gewindes und die proximale Flanke des distal benachbarten Abschnitts des zweiten Gewindes erzeugt, während das zweite Werkzeug derart ausgebildet ist, daß es gleichzeitig die proximale Flanke des ersten Gewindes und die distale Flanke des proximal benachbarten Abschnitts des zweiten Gewindes erzeugt. Die Herstellung der Schraubpfanne gestaltet sich dabei besonders schnell und damit kostengünstig, weil die den Flanken des ersten Gewindes benachbarten und zugewandten Flanken des zweiten Gewindes nach Herstellung des ersten Gewindes nicht weiter bearbeitet werden müssen und insbesondere auch kein zusätzliches Werkzeug hierfür erforderlich ist.

Es versteht sich jedoch, daß bei anderen Varianten der Erfindung die jeweiligen Flanken des ersten bzw. zweiten Gewindes jeweils in einzelnen Arbeitsgängen durch das jeweilige Werkzeug bzw. durch unterschiedliche Werkzeuge hergestellt werden können.

Weist das erste Gewinde mehr als einen Gewindegang auf, so wird es durch entsprechend aufeinanderfolgendes Bearbeiten des Pfannenkörpers mit dem ersten und zweiten Werkzeug oder durch gleichzeitiges Bearbeiten mit einer der Gangzahl entsprechenden Anzahl über den Pfannenumfang verteilter erster und zweiter Werkzeuge hergestellt. Ist das zweite Gewinde dabei eingängig ausgebildet, so entfällt eine weitere Bearbeitung des zweiten Gewindes vollständig, da dessen proximale und distale Flanken erfindungsgemäß bereits unmittelbar bei der Herstellung des ersten Gewindes mit dem bzw. den ersten und zweiten Werkzeugen erfolgt.

Bei einer bevorzugten Variante des Verfahrens wird die Schraubpfanne zur Herstellung der restlichen Flanken des zweiten Gewindes während des ersten Teilschrittes mit wenigstens einem dritten Werkzeug bearbeitet. Das dritte Werkzeug ist dabei derart ausgebildet ist, daß es gleichzeitig die proximale Flanke eines ersten Abschnitts des zweiten Gewindes und die distale Flanke des dem ersten Abschnitt proximal benachbarten zweiten Abschnitts des zweiten Gewindes erzeugt. Die einander zugewandten Flanken benachbarter Abschnitte des zweiten Gewindes werden also in einem Arbeitsgang mit einem dritten Werkzeug gefertigt. Weist das zweite Gewinde mehr als zwei Gewindegänge auf, so wird es gleichzeitig mit der Bearbeitung durch das erste und zweite Werkzeug mit einer Anzahl von dritten Werkzeugen bearbeitet, die der um Eins verringerten Gangzahl des zweiten Gewindes entspricht. Weist das zweite Gewinde also drei Gewindegänge auf, so sind bei dieser Variante dem ersten und zweiten Werkzeug zwei dritte Werkzeuge zugeordnet. Ist das erste Gewinde dabei dann noch mehrgängig, so ist für den geschilderten Fall im wesentlichen zeitgleicher Erstellung der Gewindegänge des ersten Gewindes jedem Paar aus erstem und zweitem Werkzeug eine entsprechende Anzahl an dritten Werkzeugen zugeordnet. Bei einem zweigängigen ersten Gewinde und einem sechsgängigen zweiten Gewinde wären für den Fall einer im wesentlichen Zeitgleichen Herstellung sämtlicher Gewindegänge zwei Paare aus jeweils einem ersten, einem zweiten und zwei dritten Werkzeugen erforderlich, die dann entsprechend über den Pfannenumfang verteilt sind.

Vorzugsweise entspricht dabei die Werkzeuggeometrie des dritten Werkzeugs der Werkzeuggeometrie des ersten Werkzeugs oder des zweiten Werkzeugs, da somit die erforderliche Anzahl unterschiedlicher Werkzeuge und somit auch der Herstellungsaufwand reduziert wird.

Es versteht sich, daß die Schraubpfanne in Anbetracht der begrenzten Platzverhältnisse an ihrem Umfang nur mit einer beschränkten Anzahl von Werkzeugen gleichzeitig bearbeitet werden kann. Bei anderen vorteilhaften Ausführungen des erfindungsgemäßen Verfahrens wird die Schraubpfanne zur Herstellung der restlichen Flanken des zweiten Gewindes daher während eines zweiten Teilschrittes mit entsprechenden dritten Werkzeugen bearbeitet. Vorzugsweise werden dabei das erste und/oder zweite Werkzeug als drittes Werkzeug verwendet, wobei auch hier wieder gleichzeitig die proximale Flanke eines ersten Abschnitts des zweiten Gewindes und die distale Flanke des dem ersten Abschnitt proximal benachbarten zweiten Abschnitts des zweiten Gewindes erzeugt wird. Es versteht sich jedoch, daß bei anderen Varianten der Erfindung auch hier die jeweiligen Flanken des zweiten Gewindes jeweils in einzelnen Arbeitsgängen durch das jeweilige Werkzeug bzw. durch unterschiedliche Werkzeuge hergestellt werden können.

Besonders rationelle Varianten des erfindungsgemäßen Verfahrens zeichnen sich dadurch aus, daß das erste und zweite Werkzeug im wesentlichen dieselbe Werkzeuggeometrie aufweisen. Der erforderliche Werkzeugsatz verringert sich dadurch erheblich. Dies gilt insbesondere wenn auch das bzw. die dritten Werkzeuge noch dieselbe Werkzeuggeometrie aufweist bzw. aufweisen und somit nur Werkzeuge einer bestimmten Werkzeuggeometrie erforderlich sind.

Bei herstellungstechnisch bevorzugten Weiterbildungen des Verfahrens werden die Werkzeuge bei der Bearbeitung der Schraubpfanne durch Parallelverschiebung auf einer zur Mantelfläche im wesentlichen parallelen Bahnkurve geführt. Hierbei gestaltet sich die Führung der Werkzeuge aufgrund der einfachen, in den Axialebenen der Schraubpfanne rein translatorischen Bewegung der Werkzeuge besonders einfach.

Vorzugsweise ist der den Übergangsbereich zwischen zwei Flanken im Gewindegrund erzeugende Abschnitt des jeweiligen Werkzeuges im Axialschnitt der Schraubpfanne im wesentlichen bogenförmig, insbesondere kreisbogenförmig, ausgebildet, wodurch in einfacher Weise ohne weitere Nachbearbeitung eine hinsichtlich der Festigkeit vorteilhafte Ausrundung der Übergänge zwischen den Gewindeflanken im Gewindegrund erzielt wird. Weiter vorzugsweise wird dabei der bogenförmig ausgebildete Abschnitt der Werkzeuge jeweils im wesentlichen tangential zur Mantelfläche geführt, so daß zum einen der Gewindegrund in vorteilhafter Weise der Kontur der Mantelfläche folgt und die Gewindespitzen des ersten und zweiten Gewindes mit den erwähnten Vorteilen auf einer zur Mantelfläche parallelen ersten bzw. zweiten Fläche liegen.

Als Werkzeuge können entsprechend ausgebildete Fräser, Drehmeißel, Schleifscheiben oder dergleichen verwendet werden. Der Verwendung von Fräsern oder Drehmeißeln, unter Umständen auch deren kombinierter Verwendung, wird dabei aber aufgrund ihrer entsprechend guten Verschleißfestigkeit der Vorzug gegeben.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: einen Halbschnitt eines bevorzugten Ausführungsbeispiels der Erfindung;
- Figur 2: den Schnitt aus Figur 1 während des ersten Teilschrittes des erfindungsgemäßen Verfahrens;
- Figur 3: den Schnitt aus Figur 1 während des zweiten Teilschrittes des erfindungsgemäßen Verfahrens;
- Figur 4: einen Teilschnitt durch eine weitere Ausführung der erfindungsgemäßen Schraubpfanne.

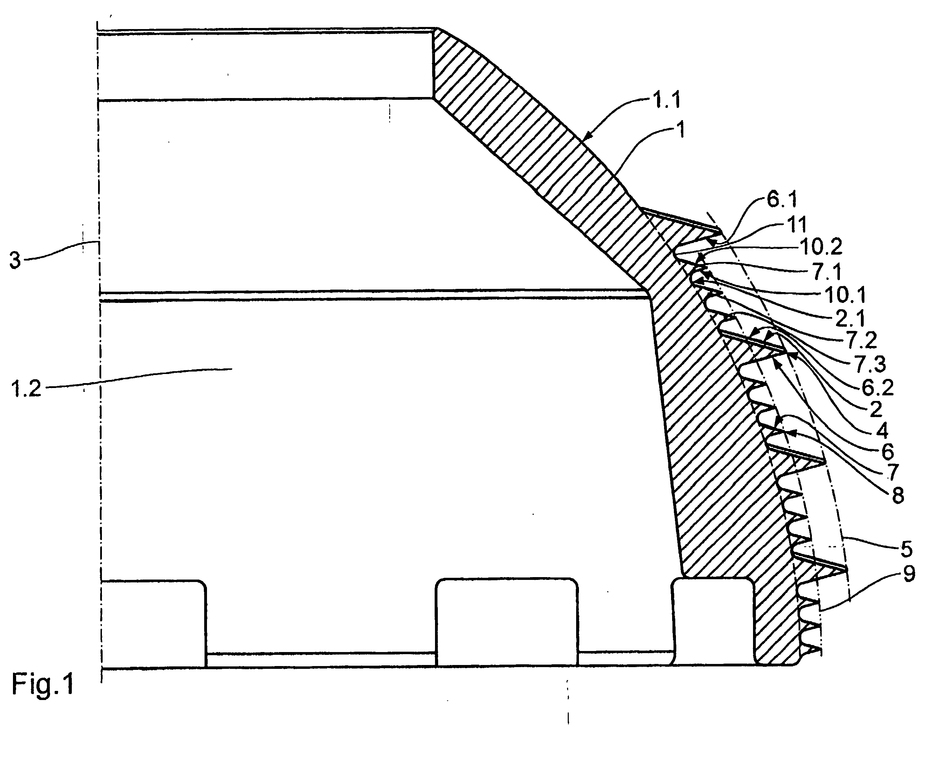

Figur 1 zeigt einen Halbschnitt durch eine Ausführung einer erfindungsgemäße Schraubpfanne mit einem Grundkörper 1 mit einer im wesentlichen halbkugelförmige Mantelfläche 1.1, auf der ein zweigängiges erstes Gewinde 2 zur zementfreien Verankerung der Schraubpfanne im Hüftknochen eines Patienten angeordnet ist. Die gezeigte Schnittebene enthält dabei die Achse 3 der Schraubpfanne. Im Innern weist der Grundkörper 1 eine Ausnehmung 1.2 zur Aufnahme eines Inlays auf, das die spätere Gleitfläche für den im Femur verankerten Kugelgelenkkopf der Hüftgelenksprothese zur Verfügung stellt.

Der Gewindegrund 2.1 des ersten Gewindes 2 liegt auf der Mantelfläche 1.1 des Grundkörpers 1, die in Figur 1 im Bereich des ersten Gewindes 2 gestrichelt dargestellt ist. Die Spitzen 4 des ersten Gewindes 2 liegen im wesentlichen über die gesamte Länge des ersten Gewindes 2 auf einer zur Mantelfläche 1.1 parallelen - in Figur 1 strichpunktiert dargestellten - Fläche 5. Die Spitzen 4 des ersten Gewindes 2 weisen somit im wesentlichen über die gesamte Länge des ersten Gewindes 2 jeweils denselben senkrechten Abstand zur Mantelfläche 1.1 auf, so daß benachbarte Flanken 6.1 und 6.2 des ersten Gewindes 2 jeweils dieselbe Flankenhöhe aufweisen. Lediglich am Ansatz und am Auslauf des ersten Gewindes 2, die beide in Figur 1 nicht dargestellt sind, verlaufen die Spitzen 4 über jeweils einen verhältnismäßig kurzen Abschnitt zwischen der Mantelfläche 1.1 und der ersten Fläche 5.

Zwischen benachbarten Flanken 6.1 und 6.2 des ersten Gewindes 2 ist im Gewindegrund 2.1 des ersten Gewindes 2 ein den Gewindegrund 2.1 im wesentlichen ausfüllendes zweites Gewinde 7 angeordnet, dessen Steigung gleich der Steigung des ersten Gewindes 2 ist. Da das erste Gewinde 2 zweigängig ausgeführt ist, gehört die Flanke 6.1 zum ersten Gewindegang des ersten Gewindes 2 und die distal benachbarte Flanke 6.2 zum zweiten Gewindegang des ersten Gewindes 2. Zwischen den beiden Flanken 6.1 und 6.2 des ersten Gewindes sind drei Gewindegänge 7.1, 7.2 und 7.3 des zweiten Gewindes 7 angeordnet. Insgesamt weist das zweite Gewinde 7 aufgrund der Zweigängigkeit des ersten Gewindes 2 die doppelte Gangzahl auf, also sechs Gewindegänge.

Die Spitzen 8 des zweiten Gewindes 7 liegen im wesentlichen über die gesamte Länge des zweiten Gewindes 7 auf einer zur Mantelfläche 1.1 parallelen - in Figur 1 strichzweipunktiert dargestellten - zweiten Fläche 9. Die Spitzen 8 des zweiten Gewindes 7 weisen somit im wesentlichen über die gesamte Länge des zweiten Gewindes 7 jeweils denselben senkrechten Abstand zur Mantelfläche 1.1 auf, so daß benachbarte Flanken 7.1 und 7.2 des zweiten Gewindes 7 jeweils dieselbe Flankenhöhe aufweisen. Lediglich am Ansatz und am Auslauf des zweiten Gewindes 7, die beide in Figur 1 nicht dargestellt sind, verlaufen die Spitzen 8, ähnlich wie schon beim ersten Gewinde 2, über jeweils einen verhältnismäßig kurzen Abschnitt zwischen der Mantelfläche 1.1 und der zweiten Fläche 9.

Die Flankenhöhe der Flanken 7.1, 7.2 und 7.3 des zweiten Gewindes 7 ist etwa um die Hälfte geringer als die Flankenhöhe der benachbarten Flanken 6.1 und 6.2 des ersten Gewindes 2. Hierdurch wird eine wesentliche Schwächung des Hüftknochens, in den die Schraubpfanne eingeschraubt wird, im Bereich zwischen den beiden benachbarten Flanken 6.1 und 6.2 des ersten Gewindes 2 durch das zweite Gewinde 7 vermieden. Der Zwischenraum im Gewindegrund 2.1 zwischen den beiden Flanken 6.1 und 6.2 des ersten Gewindes 2 ist durch die zwischen den zwei Flanken 6.1 und 6.2 des ersten Gewindes 2 angeordneten drei Gewindegänge 7.1, 7.2 und 7.3 des zweiten Gewindes 7 im wesentlichen gleichmäßig unterteilt, so daß zudem eine günstige Krafteinleitung in den Hüftknochen über das erste Gewinde 2 und das zweite Gewinde 7 sichergestellt ist.

Beide Gewinde 2 und 7 sind in bekannter Weise durch jeweils über die Gewindelänge vorgesehene Ausfräsungen selbstschneidend ausgebildet, wodurch sich eine besonders gute flächige Paarung zwischen den Gewindeflanken und dem Hüftknochen ergibt, in den die Schraubpfanne später eingeschraubt wird. Es versteht sich allerdings, daß bei anderen Ausführungen auch nur eines der beiden Gewinde selbstschneidend oder möglicherweise auch keines der beiden Gewinde selbstschneidend ausgeführt wird, wobei das entsprechende Gegengewinde dann mit entsprechenden Schneidwerkzeugen vor Einschrauben der Schraubpfanne in den Hüftknochen eingeschnitten werden muß.

Die distalen Flanken 6.1 und die proximalen Flanken 6.2 des ersten Gewindes 2 sowie die distalen Flanken 10.1 und die proximalen Flanken 10.2 des zweiten Gewindes verlaufen im in Figur 1 dargestellten Axialschnitt der Schraubpfanne um jeweils 80° zur Achse 3 der Schraubpfanne geneigt. Die distalen Flanken 6.1 des ersten Gewindes 2 sowie die distalen Flanken 10.1 des zweiten Gewindes 7 verlaufen dabei zueinander parallel, wobei sie im in Figur 1 dargestellten Axialschnitt jeweils im Uhrzeigersinn um 80° zur Achse 3 geneigt sind. Die proximalen Flanken 6.2 des ersten Gewindes 2 sowie die proximalen Flanken 10.2 des zweiten Gewindes 7 verlaufen ebenfalls zueinander parallel, wobei sie im in Figur 1 dargestellten Axialschnitt jeweils entgegen dem Uhrzeigersinn um 80° zur Achse 3 geneigt sind. Aufgrund dieser Symmetrie ergibt sich eine besonders einfache Gestaltung und Führung der die Gewinde erzeugenden Werkzeuge, mithin also eine besonders kostengünstige Herstellung der Schraubpfannen, da zur Herstellung des ersten und zweiten Gewindes ist ein und dieselbe Werkzeuggeometrie verwendet werden kann.

Der Übergangsbereich 11 im Gewindegrund 2.1 zwischen je zwei benachbarten Gewindeflanken ist, wie hier am Beispiel der distalen Flanke 6.1 des ersten Gewindes 2 und der proximalen Flanke 10.2 des ersten Gewindeganges 7.1 des zweiten Gewindes 7 gezeigt, kreisbogenförmig ausgebildet. Der Krümmungsradius des Übergangsbereichs 11 entspricht dabei der halben Flankenhöhe des zweiten Gewindes 7. Der Übergangsbereich 11 mündet dabei jeweils tangential in die distale Flanke 6.1 des ersten Gewindes 2 und die proximale Flanke 10.2 des ersten Gewindeganges 7.1 des zweiten Gewindes 7.

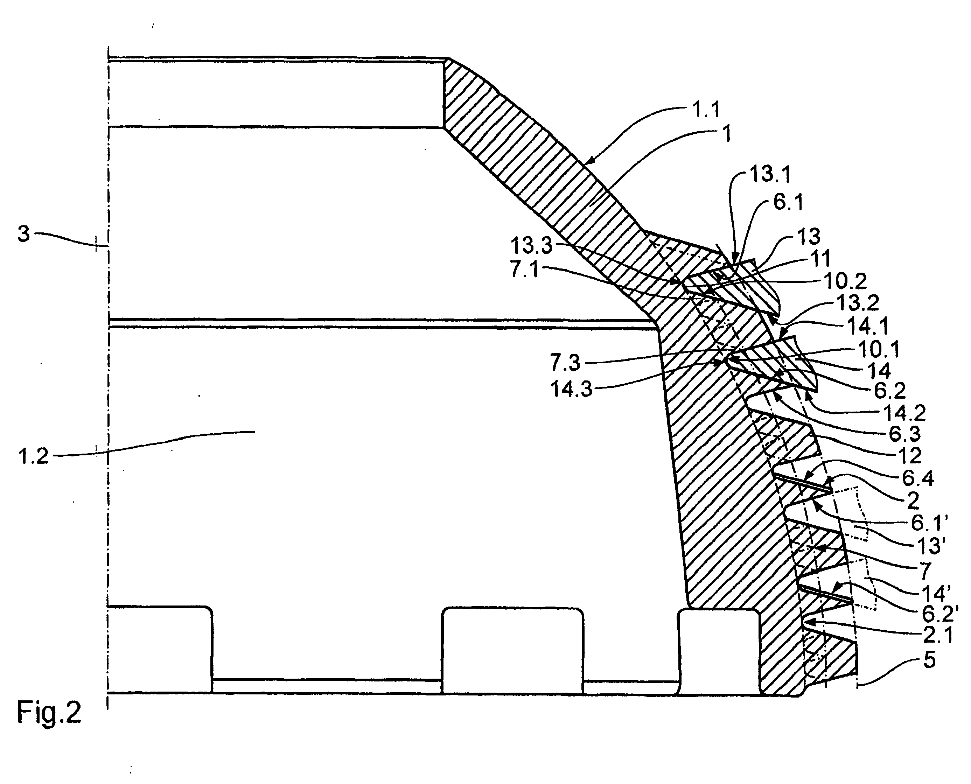

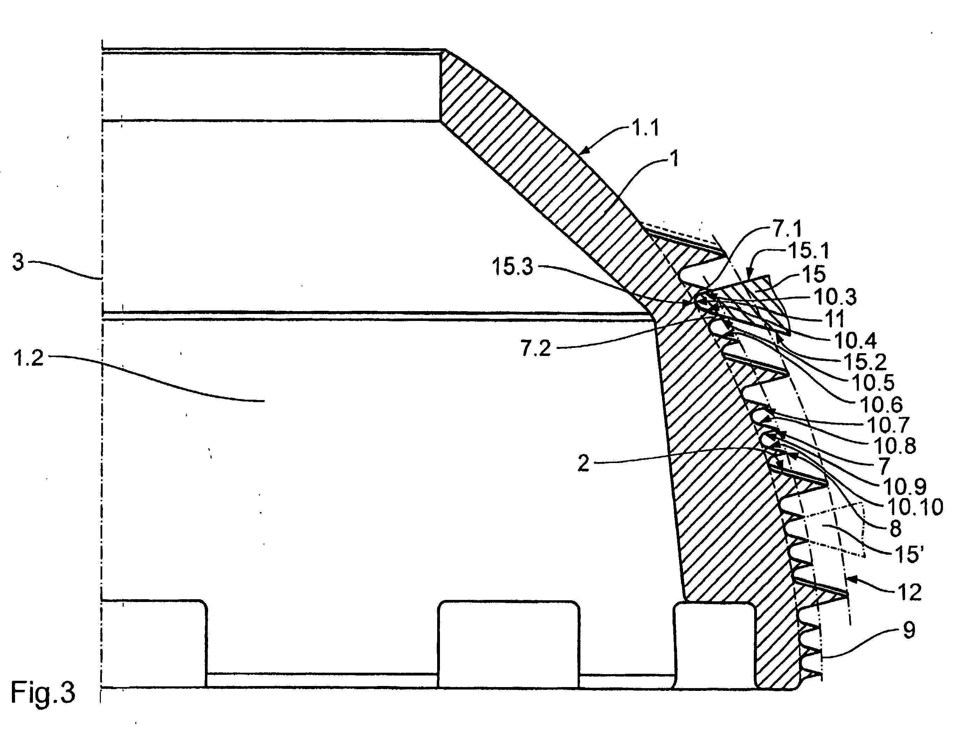

Die erfindungsgemäße Gestaltung der Schraubpfanne ermöglicht eine besonders einfache Herstellung mit dem erfindungsgemäßen Herstellungsverfahren, das im folgenden anhand der Figuren 2 und 3 erläutert wird, die jeweils einen Halbschnitt durch die Ausführung der erfindungsgemäßen Schraubpfanne aus Figur 1 zeigen, dessen Schnittverlauf dem in Figur 1 gezeigten Schnittverlauf entspricht.

Wie Figur 2 zu entnehmen ist, wird zur Herstellung des ersten Gewindes ein am Grundkörper 1 angeformter umlaufender Absatz 12 während eines ersten Teilschrittes mit einem ersten Fräser 13 und einem zweiten Fräser 14 bearbeitet. Der erste Fräser erzeugt dabei gleichzeitig die distale Flanke 6.1 des ersten Gewindes 2 und die proximale Flanke 10.2 des der distalen Flanke 6.1 distal benachbarten ersten Gewindeganges 7.1 des - in Figur 2 gestrichelt angedeuteten - zweiten Gewindes 7. Der zweite Fräser erzeugt gleichzeitig die proximale Flanke 6.2 des ersten Gewindes 2 und die distale Flanke 10.1 des der proximalen Flanke 6.2 proximal benachbarten dritten Gewindeganges 7.3 des zweiten Gewindes 7. Es versteht sich, daß anstelle von Fräsern aber auch andere Werkzeuge, wie beispielsweise Drehmeißel oder aber auch Schleifscheiben oder dergleichen verwendet werden können.

Die in Figur 2 nur im schematischen Teilschnitt gezeigten Fräser 13 und 14 sind dabei als scheibenförmige Profilfräser mit identischer Werkzeuggeometrie ausgebildet. Die Schneidkanten 13.1 und 14.1 des ersten Fräsers 13 bzw. des zweiten Fräsers 14 erzeugen dabei jeweils distale Flanken des ersten Gewindes 2 bzw. des zweiten Gewindes 7, während die Schneidkanten 13.2 und 14.2 jeweils proximale Flanken des ersten Gewindes 2 bzw. des zweiten Gewindes 7 erzeugen. Die Schneidkanten 13.1 und 14.1 des ersten Fräsers 13 bzw. des zweiten Fräsers 14 verlaufen dabei im gezeigten Axialschnitt jeweils entsprechend der Neigung der distalen Flanken des ersten Gewindes 2 bzw. des zweiten Gewindes 7 zur Schraubpfannenachse 3 geneigt. Die Schneidkanten 13.2 und 14.2 verlaufen wiederum im gezeigten Axialschnitt jeweils entsprechend der Neigung proximalen Flanken des ersten Gewindes 2 bzw. des zweiten Gewindes 7.

Der den Übergangsbereich 11 zwischen benachbarten Gewindeflanken 6.1 und 10.2 erzeugende Abschnitt 13.3 des Fräsers 13 ist entsprechend der Kontur des Übergangsbereichs 11 ebenfalls kreisbogenförmig ausgebildet. Entsprechendes gilt für den Abschnitt 14.3 des zweiten Fräsers 14.

Zur Erzeugung zueinander paralleler distaler Gewindeflanken des ersten und zweiten Gewindes und zueinander paralleler proximaler Gewindeflanken des ersten und zweiten Gewindes werden der erste Fräser 13 und der zweite Fräser 14 in der gezeigten Schnittebene auf einer zur Mantelfläche 1.1 parallelen Bahnkurve parallelverschoben, so daß ihre Schnittkanten 13.1 bzw. 13.2 und 14.1 bzw. 14.2 also stets denselben Winkel mit der Schraubpfannenachse 3 einschließen. Die Schraubpfanne wird dabei um ihre Achse 3 gedreht, so daß sie an ihrem gesamten Umfang bearbeitet wird. Die Bahnkurve des ersten Fräsers 13 bzw. zweiten Fräsers 14 verläuft dabei so, daß sie nach einer vollen Umdrehung der Schraubpfanne um die Schraubpfannenachse 3 von den - in Figur 2 gestrichelt dargestellten - Positionen 13' bzw. 14' in die gezeigte Position 13 bzw. 14 parallel verschoben wurden. Hierbei werden der erste Fräser 13 und der zweite Fräser 14 jeweils so geführt, daß der kreisbogenförmige Abschnitt 13.3 des Fräsers 13 und der kreisbogenförmige Abschnitt 14.3 des zweiten Fräsers 14 tangential zur Mantelfläche 1.1 des Grundkörpers 1 geführt wird, d. h. daß der jeweilige Abschnitt 13.3 bzw. 14.3 die Mantelfläche 1.1 des Grundkörpers jeweils in einem Punkt berührt. Hierdurch wird in einfacher Weise erreicht, daß der Gewindegrund 2.1 des ersten Gewindes 2 auf der Mantelfläche 1.1 liegt. Weiterhin werden die Fräser 13 und 14 so geführt, daß die Spitzen 4 des ersten Gewindes 2 auf der zur Mantelfläche 1.1 parallelen ersten Fläche 5 liegen.

Die Drehachsen des ersten und zweiten Fräsers 13 und 14 verlaufen - was in Figur 1 aus Gründen der Übersichtlichkeit nicht dargestellt ist - entsprechend der Gewindesteigung des ersten und zweiten Gewindes 2 und 7 zur Schnittebene geneigt, wobei sie in einer zur Schnittebene senkrechten Ebene liegen.

Da das erste Gewinde 2 zweigängig ausgeführt ist, ist zur zeitgleichen Herstellung der Flanken 6.3 und 6.4 während des ersten Teilschrittes ein - in Figur 2 nicht dargestelltes - hinsichtlich der Achse 3 zum ersten und zweiten Fräser 13 und 14 symmetrisch angeordnetes und ausgebildetes, zweites Fräserpaar vorgesehen. Es versteht sich allerdings, daß bei anderen Varianten zur Herstellung der Flanken 6.3 und 6.4 auch in einem zweiten Arbeitsgang während des ersten Teilschrittes die Fräser 13 und 14 verwendet werden könnten.

Wie Figur 3 zu entnehmen ist, werden in einem zweiten Teilschritt der beschriebenen Ausführung des erfindungsgemäßen Verfahrens die restlichen Flanken des zweiten Gewindes 7 durch Bearbeitung des Absatzes 12 erzeugt. Hierzu ist ein dritter Fräser 15 vorgesehen, der dieselbe Werkzeuggeometrie wie der erste und zweite Fräser 13 und 14 aufweist. Auch die Winkellage der Schneidkanten 15.1 bzw. 15.2 des dritten Fräsers 15 hinsichtlich der Achse 3 entspricht der beschriebenen Winkellage der Schneidkanten 13.1 bzw. 13.2 des ersten Fräsers 13. Gleiches gilt für die Ausrichtung der Fräserachse zur Schnittebene.

Der dritte Fräser erzeugt gleichzeitig die distale Flanke 10.3 des ersten Gewindeganges 7.1 des zweiten Gewindes 7 und die proximale Flanke 10.4 des der Flanke 10.3 distal benachbarten zweiten Gewindeganges 7.2 des zweiten Gewindes 7. Es versteht sich, daß auch hier anstelle des Fräsers ein anderes Werkzeug, wie beispielsweise ein Drehmeißel oder aber auch eine Schleifscheibe oder dergleichen verwendet werden kann.

Die Arbeitsweise des dritten Fräsers 15 während des zweiten Teilschrittes gleicht derjenigen des ersten Fräsers 13 während des ersten Teilschrittes. Der dritte Fräser 15 wird ebenfalls auf einer zur Mantelfläche 1.1 parallelen Bahn durch Parallelverschiebung in der gezeigten Schnittebene verfahren. Während einer Umdrehung der Schraubpfanne wird der dritte Fräser 15 von der - in Figur 3 gestrichelt dargestellten - Position 15' in die Position 15 verfahren, wobei sein kreisbogenförmiger Schneidkantenabschnitt 15.3 tangential zur Mantelfläche 1.1 geführt wird. Hierdurch wird in einfacher Weise erreicht, daß der Gewindegrund 2.1 des ersten Gewindes 2 auf der Mantelfläche 1.1 liegt. Weiterhin wird der Fräser 15 so geführt, daß die Spitzen 8 des zweiten Gewindes 7 auf der zur Mantelfläche 1.1 parallelen ersten Fläche 9 liegen.

Zur zeitgleichen Herstellung der restlichen Flanken 10.5 bis 10.10 des zweiten Gewindes 7 sind im beschriebenen Beispiel - in Figur 3 nicht gezeigte - drei weitere dritte Fräser am Umfang der Schraubpfanne verteilt vorgesehen, die dem dritten Fräser 15 in Geometrie und Verwendung gleichen. Es versteht sich jedoch, daß bei anderen Varianten des Verfahrens auch eine geringere Anzahl weiterer dritter Fräser vorgesehen sein kann, die die restlichen Flanken des zweiten Gewindes dann in mehreren Arbeitsgängen während des zweiten Teilschrittes erzeugen. Insbesondere kann auch nur ein dritter Fräser vorgesehen sein, der dann die restlichen Flanken des zweiten Gewindes in vier Arbeitsgängen während des zweiten Teilschrittes erzeugt. Es versteht sich weiterhin, daß infolge der identischen Werkzeuggeometrie als dritter bzw. dritte Fräser auch der erste oder zweite Fräser verwendet werden können.

Im beschriebenen Beispiel des Verfahrens wurden das erste Gewinde und die restlichen Flanken des zweiten Gewindes in zwei aufeinanderfolgenden Teilschritten erzeugt. Bei anderen Varianten des erfindungsgemäßen Verfahrens kann die Herstellung des ersten und zweiten Gewindes jedoch auch bei entsprechender Anordnung der Werkzeuge am Umfang der Schraubpfanne im wesentlichen zeitgleich während des ersten Teilschrittes erfolgen.

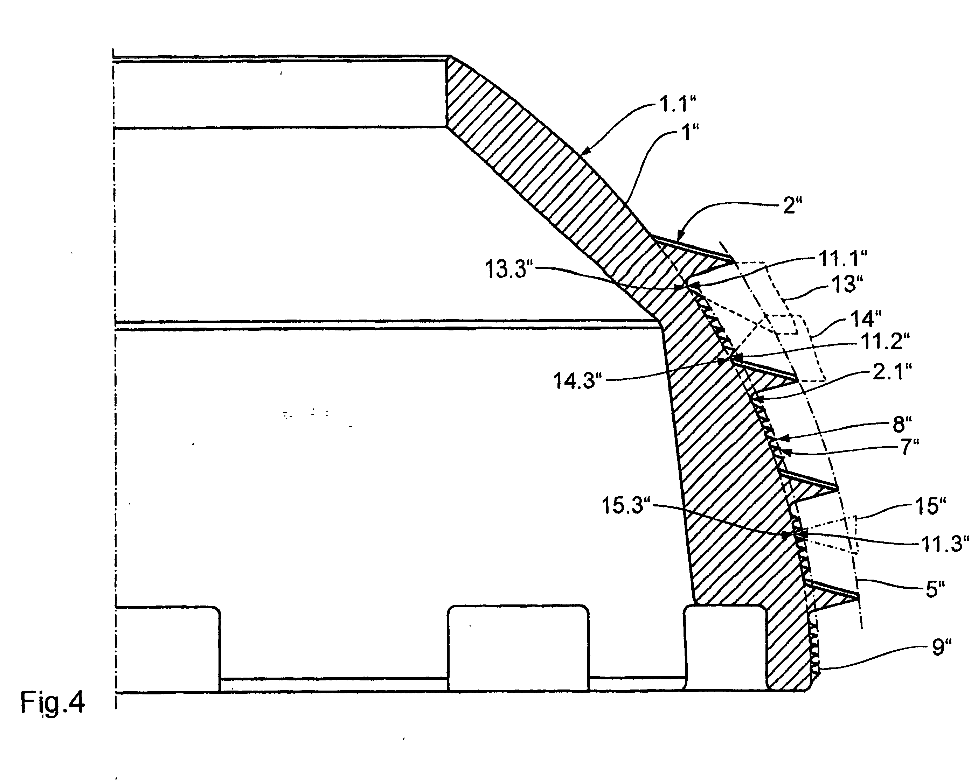

Figur 4 zeigt einen Axialteilschnitt durch eine weitere Ausführung der erfindungsgemäßen Schraubpfanne. Aufbau und Herstellung dieser Variante entsprechen im wesentlichen der zu den Figuren 1 bis 3 beschriebenen Ausführung, so daß hier lediglich auf die Unterschiede zu dieser Ausführung eingegangen werden soll.

Das erste Gewinde 2" entspricht im wesentlichen dem ersten Gewinde aus Figur 1, während das zweite Gewinde 7" eine bedeutend geringere Flankenhöhe aufweist als das zweite Gewinde aus Figur 1. Die Flankenhöhe des zweiten Gewindes 7" entspricht dabei etwa einem Sechstel der Flankenhöhe des ersten Gewindes 2". Das zweite Gewinde 7" ist im gezeigten Beispiel nicht selbstschneidend ausgebildet. Die Flankenhöhe des zweiten Gewindes 7" ist jedoch so klein und die Gewindezähne sind so schlank, daß die Flanken des zweiten Gewindes 7" beim Einschrauben der Schraubpfanne in eine Aussparung im Hüftknochen gegebenenfalls problemlos in den Hüftknochen gedrückt werden. Der Kontakt zwischen den Gewindespitzen 8" und dem Hüftknochen regt diesen dann zum Wachstum an, so daß Knochengewebe in die Gewindegänge des zweiten Gewindes 7" einwächst.

Die Herstellung der in Figur 4 dargestellten Ausführung der Schraubpfanne entspricht im wesentlichen dem zu den Figuren 2 und 3 geschilderten Herstellungsverfahren. Der Unterschied besteht lediglich darin, daß das erste Werkzeug 13", das zweite Werkzeug 14" und das dritte Werkzeug 15" - deren Kontur in Figur 4 jeweils gestrichelt angedeutet ist - jeweils unterschiedliche Werkzeuggeometrie aufweisen. Zudem sind das erste Werkzeug 13" und das zweite Werkzeug 14" aufgrund ihrer Überdeckung zueinander in Umfangsrichtung der Schraubpfanne versetzt angeordnet.

Das erste Werkzeug 13" bzw. das zweite Werkzeug 14" weist an seinem jeweiligen Spitzenabschnitt 13.3" bzw. 14.3" einen größeren Schneidkantenradius auf als das dritte Werkzeug 15" an seinem Spitzenabschnitt 15.3". Die Ausrundungsradien in den Übergangsbereichen 11.1'' und 11.2" zwischen je einer Flanke des ersten Gewindes 2" und des zweiten Gewindes 7" sind daher ebenfalls größer als die Ausrundungsradien in den Übergangsbereichen 11.3'' zwischen je zwei Flanken des zweiten Gewindes 7''. Als Werkzeuge 13", 14" und 15" werden im in Figur 4 gezeigten Beispiel einfache Drehmeißel verwendet.

Es versteht sich, daß bei anderen Ausführungen durch entsprechend groß gewählten Schneidkantenradius am Spitzenabschnitt des dritten Werkzeugs und entsprechender Führung des dritten Werkzeugs ohne weiters auch noch kleinere Flankenhöhen des zweiten Gewindes und zusätzlich oder alternativ eine geringere Gangzahl des zweiten Gewindes erzielt werden kann.

Die Erfindung beschränkt sich in ihrer Ausführung nicht auf die vorstehend angegebenen bevorzugten Ausführungsbeispiele. Vielmehr ist eine Anzahl von Varianten möglich, welche von der dargestellten Lösung auch bei grundsätzlich anders gearteten Ausführungen Gebrauch macht.

## Patentansprüche

1. Schraubpfanne für eine Hüftgelenksprothese mit einem Grundkörper (1; 1''), der eine zur Pfannenachse (3) im wesentlichen rotationssymmetrische, insbesondere im wesentlichen halbkugelförmige, ellipsoidförmige oder konische Mantelfläche (1.1; 1.1") aufweist, und einem auf der Außenseite des Grundkörpers (1; 1") angeordneten ersten Gewinde (2; 2") zur Verankerung der Schraubpfanne im Hüftknochen, dessen Gewindegrund (2.1; 2.1") im wesentlichen auf der Mantelfläche (1.1; 1.1'') des Grundkörpers (1; 1") liegt, wobei
zwischen benachbarten Flanken (6.1, 6.2) des ersten Gewindes (2; 2'') wenigstens ein den Gewindegrund (2.1; 2.1") des ersten Gewindes (2; 2") im wesentlichen ausfüllendes zweites Gewinde (7; 7'') vorgesehen ist, dessen lokale Flankenhöhe senkrecht zur Mantelfläche (1.1; 1.1") über im wesentlichen die gesamte Länge des zweiten Gewindes (7; 7'') wesentlich geringer ist als die lokale Flankenhöhe der beiden benachbarten Flanken (6.1, 6.2) oder der kürzeren der beiden benachbarten Flanken (6.1, 6.2) des ersten Gewindes (2; 2"),
**dadurch gekennzeichnet daß** das erste Gewinde (2; 2") und/oder das zweite Gewinde (7; 7") mehrgängig ausgebildet ist bzw. sind und die Gangzahl des zweiten Gewindes (7; 7") einem ganzzahligen Vielfachen der Gangzahl des ersten Gewindes (2; 2") entspricht.

2. Schraubpfanne nach Anspruch 1, **dadurch gekennzeichnet, daß** die lokale Flankenhöhe des zweiten Gewindes (2; 2") um wenigstens die Hälfte, vorzugsweise wenigstens zwei Drittel, geringer ist als die lokale Flankenhöhe der beiden benachbarten Flanken (6.1, 6.2) oder der kürzeren der beiden benachbarten Flanken (6.1, 6.2) des ersten Gewindes (7; 7").

3. Schraubpfanne nach Anspruch 1 oder 2, **dadurch gekennzeichnet, daß** das erste Gewinde (2; 2") und/oder das zweite Gewinde (7; 7'') selbstschneidend ausgebildet ist.

4. Schraubpfanne nach einem der vorhergehenden Ansprüche, **dadurch gekennzeichnet, daß** das erste Gewinde (2; 2") zweigängig und das zweite Gewinde (7; 7'') wenigstens viergängig ausgebildet ist.

5. Schraubpfanne nach einem der vorhergehenden Ansprüche, **dadurch gekennzeichnet, daß** das zweite Gewinde (7; 7'') derart ausgebildet und angeordnet ist, daß der Zwischenraum im Gewindegrund (2.1; 2.1") zwischen zwei Flanken (6.1, 6.2) des ersten Gewindes im Axialschnitt der Schraubpfanne durch den zwischen den zwei Flanken (6.1, 6.2) des ersten Gewindes (2; 2") angeordneten Gewindegang des zweiten Gewindes (7; 7") oder die zwischen den zwei Flanken (6.1, 6.2) des ersten Gewindes angeordneten Gewindegänge des zweiten Gewindes (7; 7'') im wesentlichen gleichmäßig unterteilt ist.

6. Schraubpfanne nach einem der vorhergehenden Ansprüche, **dadurch gekennzeichnet, daß** der Übergangsbereich (11; 11.1", 11.2", 11.3") zwischen zwei Flanken (6.1, 10.2; 10.3, 10.4; 10.1, 6.2) im Gewindegrund (2.1; 2.1") im Axialschnitt der Schraubpfanne im wesentlichen bogenförmig ausgebildet ist.

7. Schraubpfanne nach Anspruch 6, **dadurch gekennzeichnet, daß** der Übergangsbereich (11) im Axialschnitt der Schraubpfanne im wesentlichen kreisbogenförmig ausgebildet ist, wobei der Übergangsbereich (11) jeweils im wesentlichen tangential in eine der beiden Flanken (6.1, 10.2; 10.3, 10.4; 10.1, 6.2) mündet und einen Krümmungsradius aufweist, der höchstens im wesentlichen der halben Flankenhöhe des zweiten Gewindes (7) entspricht.

8. Schraubpfanne nach einem der vorhergehenden Ansprüche, **dadurch gekennzeichnet, daß** die distalen Flanken (6.1, 6.3) des ersten Gewindes (2; 2") und die distalen Flanken (10.1, 10.3, 10.5, 10.7, 10.9) des zweiten Gewindes (7; 7") im Axialschnitt der Schraubpfanne zur Achse (3) der Schraubpfanne um einen Winkel von im wesentlichen 70° bis 90°, vorzugsweise 75° bis 85°, geneigt verlaufen sowie die proximalen Flanken (6.2, 6.4) des ersten Gewindes (2; 2") und die proximalen Flanken (10.2, 10.4, 10.6, 10.8, 10.10) des zweiten Gewindes (7; 7") im Axialschnitt der Schraubpfanne zur Achse (3) der Schraubpfanne um einen Winkel von im wesentlichen 70° bis 90°, vorzugsweise 75° bis 85°, geneigt verlaufen.

9. Schraubpfanne nach Anspruch 8, **dadurch gekennzeichnet, daß** die distalen und proximalen Flanken (6.1, 6.3; 6.2, 6.4) des ersten Gewindes (2; 2") im Axialschnitt der Schraubpfanne zur Achse (3) der Schraubpfanne um betragsmäßig im wesentlichen denselben Winkel geneigt verlaufen sowie die distalen und proximalen Flanken (10.1, 10.3, 10.5, 10.7, 10.9; 10.2, 10.4, 10.6, 10.8, 10.10) des zweiten Gewindes (7; 7'') im Axialschnitt der Schraubpfanne zur Achse (3) der Schraubpfanne um betragsmäßig im wesentlichen denselben Winkel geneigt verlaufen.

10. Schraubpfanne nach Anspruch 8 oder 9, **dadurch gekennzeichnet, daß** die distalen Flanken (6.1, 6.3) des ersten Gewindes (2) und die distalen Flanken (10.1, 10.3, 10.5, 10.7, 10.9) des zweiten Gewindes (7) sowie die proximalen Flanken (6.2, 6.4) des ersten Gewindes (2) unddie proximalen Flanken (10.2, 10.4, 10.6, 10.8, 10.10) des zweiten Gewindes (7) zueinander im wesentlichen parallel verlaufen.

11. Schraubpfanne nach einem der vorhergehenden Ansprüche, **dadurch gekennzeichnet, daß** die Spitzen (4) des ersten Gewindes (2; 2") über im wesentlichen die gesamte Länge des ersten Gewindes (2; 2") auf einer zur Mantelfläche (1.1; 1.1'') des Grundkörpers (1; 1'') im wesentlichen parallelen ersten Fläche (5; 5") liegen und/oder die Spitzen (8) des zweiten Gewindes (7; 7") über im wesentlichen die gesamte Länge des zweiten Gewindes (7; 7'') auf einer zur Mantelfläche (1.1; 1.1'') des Grundkörpers (1; 1'') im wesentlichen parallelen zweiten Fläche (9; 9") liegen.

12. Schraubpfanne nach Anspruch 11, **dadurch gekennzeichnet, daß** die Mantelfläche (1.1; 1.1'') im wesentlichen halbkugelförmig ist.

13. Verfahren zur spanenden Herstellung einer Schraubpfanne nach einem der vorhergehenden Ansprüche, **dadurch gekennzeichnet, daß** die Schraubpfanne in einem ersten Teilschritt zur Erzeugung des ersten Gewindes (2; 2") mit wenigstens einem ersten Werkzeug (13; 13") und einem zweiten Werkzeug (14; 14") bearbeitet wird und während des ersten Teilschrittes oder in einem zweiten Teilschritt die restlichen Flanken (10.3, 10.4, 10.5, 10.6) des zweiten Gewindes (7; 7'') ausgebildet werden.

14. Verfahren nach Anspruch 13, **dadurch gekennzeichnet, daß** das erste Werkzeug (13; 13") derart ausgebildet ist, daß es gleichzeitig die distale Flanke (6.1) des ersten Gewindes (2; 2") und die proximale Flanke (10.2) des distal benachbarten Abschnitts (7.1) des zweiten Gewindes (1; 1'') erzeugt, und das zweite Werkzeug (14; 14") derart ausgebildet ist, daß es gleichzeitig die proximale Flanke (6.2) des ersten Gewindes (2; 2") und die distale Flanke (10.1) des proximal benachbarten Abschnitts (7.3) des zweiten Gewindes (7; 7") erzeugt.

15. Verfahren nach Anspruch 13 oder 14, **dadurch gekenn** **zeichnen,** daß die Schraubpfanne zur Herstellung der restlichen Flanken (10.3, 10.4, 10.5, 10.6) des zweiten Gewindes (7; 7'') während des ersten Teilschrittes mit wenigstens einem dritten Werkzeug (15; 15") bearbeitet wird, das derart ausgebildet ist, daß es gleichzeitig die proximale Flanke (10.4) eines ersten Abschnitts (7.2) des zweiten Gewindes (7; 7'') und die distale Flanke (10.3) des dem ersten Abschnitt (7.2) proximal benachbarten zweiten Abschnitts (7.1) des zweiten Gewindes (7; 7") erzeugt.

16. Verfahren nach Anspruch 15, **dadurch gekennzeichnet, daß** die Werkzeuggeometrie des dritten Werkzeugs (15; 15") der Werkzeuggeometrie des ersten Werkzeugs (13; 13") oder des zweiten Werkzeugs (14; 14") entspricht.

17. Verfahren nach Anspruch 13 oder 14, **dadurch gekennzeichnet, daß** die Schraubpfanne zur Herstellung der restlichen Flanken (10.3, 10.4, 10.5, 10.6) des zweiten Gewindes (7) während des zweiten Teilschrittes mit dem ersten Werkzeug (13) und/oder dem zweiten Werkzeug (14) derart bearbeitet wird, daß gleichzeitig die proximale Flanke (10.4) eines ersten Abschnitts (7.2) des zweiten Gewindes (7; 7'') und die distale Flanke (10.3) des dem ersten Abschnitt (7.2) proximal benachbarten zweiten Abschnitts (7.1) des zweiten Gewindes (7) erzeugt wird.

18. Verfahren nach einem der Ansprüche 13 bis 17, **dadurch gekennzeichnet, daß** das erste und zweite Werkzeug (13, 14; 13", 14") im wesentlichen dieselbe Werkzeuggeometrie aufweisen.

19. Verfahren nach einem der Ansprüche 13 bis 18, **dadurch gekennzeichnet, daß** die Werkzeuge (13, 14, 15; 13", 14", 15") bei der Bearbeitung der Schraubpfanne durch Parallelverschiebung auf einer zur Mantelfläche (1.1; 1.1") im wesentlichen parallelen Bahnkurve geführt werden.

20. Verfahren nach einem der Ansprüche 13 bis 19, **dadurch gekennzeichnet, daß** der den Übergangsbereich (11) zwischen zwei Flanken (6.1, 10.2; 10.3, 10.4; 10.1, 6.2) im Gewindegrund (2.1; 2.1") erzeugende Abschnitt (13.3, 14.3, 15.3; 13.3", 14.3", 15.3") des jeweiligen Werkzeuges (13, 14, 15; 13", 14", 15") im Axialschnitt der Schraubpfanne im wesentlichen bogenförmig, insbesondere kreisbogenförmig, ausgebildet ist.

21. Verfahren nach Anspruch 20, **dadurch gekennzeichnet, daß** der bogenförmig ausgebildete Abschnitt (13.3, 14.3, 15.3; 13.3", 14.3", 15.3") der Werkzeuge (13, 14, 15; 13", 14", 15") jeweils im wesentlichen tangential zur Mantelfläche (1.1; 1.1") geführt wird.

22. Verfahren nach einem der Ansprüche 13 bis 21, **dadurch gekennzeichnet, daß** als Werkzeuge (13, 14, 15; 13", 14", 15'') entsprechend ausgebildete Fräser und/oder Drehmeißel verwendet werden.

## Claims

1. Threaded acetabular cup for a hip joint prosthesis with a main body (1; 1'') which has a lateral area (1.1; 1.1'') essentially rotationally symmetrical to the cup axis and which is particularly essentially hemispherical, ellipsoidal or conical and with a first thread (2; 2'') arranged on the outer side of the main body (1; 1'') for anchoring the threaded acetabular cup in the hip bone wherein the thread base (2.1; 2.1'') lies essentially on the lateral area (1.1; 1.1'') of the main body (1; 1'') wherein between adjacent flanks (6.1, 6.2) of the first thread (2; 2'') at least a second thread (7; 7") essentially filling out the thread base (2.1; 2.1'') of the first thread (2; 2'') is provided whose local flank height perpendicular to the lateral area (1.1; 1.1'') over essentially the entire length of the second thread (7; 7'') is considerably smaller than the local flank height of the two adjacent flanks (6.1, 6.2) or of the shorter of the two adjacent flanks (6.1, 6.2) of the first thread (2; 2")
**characterised in that**
the first thread (2; 2") and/or the second thread (7; 7'') is / are formed with multiple screws and the number of screws of the second thread (7; 7'') corresponds to a whole multiple of the number of screws of the first thread (2; 2'').

2. Threaded acetabular cup according to claim 1, **characterised in that** the local flank height of the second thread (2; 2") is smaller by at least half, preferably at least two thirds, than the local flank height of the two adjacent flanks (6.1, 6.2) or of the shorter of the two adjacent flanks (6.1, 6.2) of the first thread (7; 7'').

3. Threaded acetabular cup according to claim 1 or 2, **characterised in that** the first thread (2; 2'') and/or the second thread (7; 7'') is in self-tapping form.

4. Threaded acetabular cup according to one of the preceding claims, **characterised in that** the first thread (2; 2'') is a two screw thread and the second thread (7; 7'') is at least a four screw thread.

5. Threaded acetabular cup according to one of the preceding claims, **characterised in that** the second thread (7; 7'') is formed and arranged in such a way that the intermediate space in the thread base (2.1; 2.1'') between two flanks (6.1, 6.2) of the first thread in the axial section of the threaded acetabular cup is sub-divided essentially evenly by the thread screw of the second thread (7; 7'') arranged between the two flanks (6.1, 6.2) of the first thread (2; 2'') or the thread screws of the second thread (7; 7'') arranged between the two flanks (6.1, 6.2) of the first thread.

6. Threaded acetabular cup according to one of the preceding claims, **characterised in that** the transient area (11; 11.1'', 11.2'', 11.3'') between two flanks (6.1, 10.2; 10.3, 10.4; 10.1, 6.2) in the thread base (2.1; 2.1'') in the axial section of the threaded acetabular cup is in an essentially arc form.

7. Threaded acetabular cup according to claim 6, **characterised in that** the transient area (11) in the axial section of the threaded acetabular cup is formed essentially as the arc of a circle wherein the transient area (11) runs essentially tangentially into one of the two flanks (6.1, 10.2; 10.3, 10.4; 10.1, 6.2) and has a radius of curvature which corresponds at the most essentially to half the flank height of the second thread (7).

8. Threaded acetabular cup according to one of the preceding claims, **characterised in that** the distal flanks (6.1, 6.3) of the first thread (2; 2'';) and the distal flanks (10.1, 10.3, 10.5, 10.7, 10.9) of the second thread (7; 7") in the axial section of the threaded acetabular cup are inclined to the axis (3) of the threaded acetabular cup by an angle of essentially 70° to 90°, preferably 75° to 85°, and the proximal flanks (6.2, 6.4) of the first thread (2; 2'') and the proximal flanks (10.2, 10.4, 10.6, 10.8, 10.10) of the second thread (7; 7'') in the axial section of the threaded acetabular cup are inclined to the axis (3) of the threaded acetabular cup by an angle of essentially 70° to 90°, preferably 75° to 85°.

9. Threaded acetabular cup according to claim 8, **characterised in that** the distal and proximal flanks (6.1, 6.3; 6.2, 6.4) of the first thread (2; 2'') in the axial section of the threaded acetabular cup are inclined to the axis (3) of the threaded acetabular cup by, in terms of amount, essentially the same angle and the distal and proximal flanks (10.1, 10.3, 10.5, 10.7, 10.9; 10.2, 10.4, 10.6, 10.8, 10.10) of the second thread (7; 7'') in the axial section of the threaded acetabular cup are inclined to the axis (3) of the threaded acetabular cup by, in terms of amount, essentially the same angle.

10. Threaded acetabular cup according to claim 8 or 9, **characterised in that** the distal flanks (6.1, 6.3) of the first thread (2) and the distal flanks (10.1, 10.3, 10.5, 10.7, 10.9) of the second thread (7) as well as the proximal flanks (6.2, 6.4) of the first thread (2) and the proximal flanks (10.2, 10.4, 10.6, 10.8, 10.10) of the second thread (7) are essentially parallel to each other.

11. Threaded acetabular cup according to one of the preceding claims, **characterised in that** the points (4) of the first thread (2; 2'') over essentially the entire length of the first thread (2; 2'') lie on a first area (5; 5'') essentially parallel to the lateral area (1.1; 1.1'') of the main body (1; 1'') and/or the points (8) of the second thread (7; 7'') over essentially the entire length of the second thread (7; 7'') lie on a second area (9' 9'') essentially parallel to the lateral area (1.1; 1.1'') of the main body (1; 1'').

12. Threaded acetabular cup according to claim 11, **characterised in that** the lateral area (1.1; 1.1'') is in essentially hemispherical form.

13. Method of manufacture by machine of a threaded acetabular cup according to one of the preceding claims, **characterised in that** the threaded acetabular cup is processed in a first partial step for the production of the first thread (2; 2'') with at least a first tool (13; 13'') and a second tool (14; 14'') and during the first partial step or in a second partial step the remaining flanks (10.3, 10.4, 10.5, 10.6) of the second thread (7; 7'') are formed.

14. Method according to claim 13, **characterised in that** the first tool (13; 13'') is formed in such a way that it simultaneously produces the distal flank (6.1) of the first thread (2; 2'') and the proximal flank (10.2) of the distally adjacent section (7.1) of the second thread (1; 1'') and the second tool (14; 14'') is formed in such a way that it simultaneously produces the proximal flank (6.2) of the first thread (2; 2'') and the distal flank (10.1) of the proximally adjacent section (7.3) of the second thread (7; 7'').

15. Method according to claim 13 or 14, **characterised in that** the threaded acetabular cup for the purpose of manufacture of the remaining flanks (10.3, 10.4, 10.5, 10.6) of the second thread (7; 7'') during the first partial step is processed with at least a third tool (15; 15'') which is formed in such a way that it simultaneously produces the proximal flank (10.4) of a first section (7.2) of the second thread (7; 7'') and the distal flank (10.3) of the second section (7.1) of the second thread (7; 7'') proximally adjacent to the first section (7.2).

16. Method according to claim 15, **characterised in that** the tool geometry of the third tool (15; 15'') corresponds to the tool geometry of the first tool (13; 13'') or of the second tool (14; 14'').

17. Method according to claim 13 or 14, **characterised in that** the threaded acetabular cup for the purpose of manufacture of the remaining flanks (10.3, 10.4, 10.5, 10.6) of the second thread (7) during the second partial step is processed with the first tool (13) and/or the second tool (14) in such a way that the proximal flank (10.4) of a first section (7.2) of the second thread (7; 7'') and the distal flank (10.3) of the second section (7.1) of the second thread (7) proximally adjacent to the first section (7.2) are produced simultaneously.

18. Method according to one of the claims 13 to 17, **characterised in that** the first and second tool (13, 14; 13'', 14'') have essentially the same tool geometry.

19. Method according to one of the claims 13 to 18, **characterised in that** the tools (13, 14, 15; 13'', 14'', 15'') in the processing of the threaded acetabular cup are guided through parallel displacement on a trajectory curve essentially parallel to the lateral area (1.1; 1.1'').

20. Method according to one of the claims 13 to 19, **characterised in that** the section (13.3, 14.3, 15.3; 13.3'', 14.3, 15.3'') of the respective tool (13, 14, 15; 13'', 14'', 15'') producing the transient area (11) between two flanks (6.1, 10.2; 10.3, 10.4; 10.1, 6.2) in the thread base (2.1; 2.1'') is formed in the axial section of the threaded acetabular cup in an essentially arc form, in particular the arc of a circle.

21. Method according to claim 20, **characterised in that** the section (13.3, 14.3, 15.3; 13.3'', 14.3'', 15.3'') of the tools (13, 14, 15; 13'', 14", 15'') formed as an arc is guided respectively essentially tangentially to the lateral area (1.1; 1.1").

22. Method according to one of the claims 13 to 21, **characterised in that** correspondingly formed milling cutters and/or lathe tools are used as tools (13, 14, 15'; 13'', 14'', 15'').

## Revendications

1. Coque acétabulaire filtrée pour une prothèse de l'articulation de la hanche, avec un corps de base (1; 1") présentant, une surface de gainage (1.1; 1.1") sensiblement à symétrie de révolution autour de l'axe de la coque (3) , en particulier sensiblement de forme hémisphérique, de forme ellipsoïdale ou conique, et avec un premier filetage (2; 2") et disposé sur la face externe du corps de base (1; 1") pour la fixation de la coque acétabulaire dans l'os iliaque, dont le fond de filet (2.1; 2.1") se trouve sensiblement sur la surface de gainage (1.1; 1.1") du corps de base (1; 1"), et telle qu'entre des flancs contigus (6.1; 6.2) du premier filetage (2; 2"), au moins un deuxième filetage (7; 7") est prévu, remplissant sensiblement le fond de filet (2.1; 2.1") du premier filetage (2; 2"), dont la hauteur locale des flancs, verticalement à la surface de gainage (1.1; 1.1"), sur sensiblement toute la longueur du deuxième filetage (7; 7"), est sensiblement plus faible que la hauteur locale des flancs des deux flancs contigus (6.1; 6.2) ou du plus court des deux flancs contigus (6.1; 6.2) du premier filetage (2; 2"), **caractérisée en ce que** le premier filetage (2; 2") et/ou le deuxième filetage (7; 7") est ou sont configuré(s) avec des filetages multiples et **en ce que** le nombre de filets du deuxième filetage (7; 7") correspond à un multiple entier du nombre de filets du premier filetage (2; 2").

2. Coque acétabulaire selon la revendication 1, **caractérisée en ce que** la hauteur locale des flancs du deuxième filetage (7; 7") est d'au moins la moitié, de préférence d'au moins deux tiers, plus faible que la hauteur locale des flancs des deux flancs contigus (6.1; 6.2) ou du plus court des deux flancs contigus (6.1; 6.2) du premier filetage (2; 2").

3. Coque acétabulaire selon la revendication 1 ou 2, **caractérisée en ce que** le premier filetage (2; 2") et/ou le deuxième filetage (7; 7") ont une configuration autotaraudeuse.

4. Coque acétabulaire selon l'une des revendications précédentes, **caractérisée en ce que** le premier filetage (2; 2") est configuré en deux filets et le deuxième filetage (7; 7") au moins en quatre filets.

5. Coque acétabulaire selon l'une des revendications précédentes, **caractérisée en ce que** le deuxième filetage (7; 7") est configuré et disposé de telle sorte que l'espace intermédiaire dans le fond de filet (2.1; 2.1") entre deux flancs (6.1; 6.2) du premier filetage, en coupe axiale de la coque acétabulaire, est divisé sensiblement de manière régulière par le pas de filet du deuxième filetage (7; 7") disposé entre les deux flancs (6.1; 6.2) du premier filetage (2; 2") ou par les pas de filet du deuxième filetage (7; 7") disposés entre les deux flancs (6.1; 6.2) du premier filetage.

6. Coque acétabulaire selon l'une des revendications précédentes, **caractérisée en ce que** la zone de transition (11; 11.1", 11.2", 11.3") est sensiblement configurée cintrée en coupe axiale de la coque acétabulaire, entre deux flancs (6.1, 10.2; 10.3, 10.4; 10.1, 6.2) dans le fond de filet (2.1; 2.1").

7. Coque acétabulaire selon la revendication 6, **caractérisée en ce que** la zone de transition (11) est configurée sensiblement en forme d'arc de cercle, en coupe axiale de la coque acétabulaire, et telle que la zone de transition (11) aboutit chaque fois sensiblement de manière tangentielle dans l'un des deux flancs (6.1, 10.2; 10.3, 10.4; 10.1, 6.2) et présente un rayon de courbure qui est au plus sensiblement égal à la moitié de la hauteur des flancs du deuxième filetage (7).

8. Coque acétabulaire selon l'une des revendications précédentes, **caractérisée en ce que** les flancs distaux (6.1, 6.3) du premier filetage (2; 2") et les flancs distaux (10.1, 10.3, 10.5, 10.7, 10,9) du deuxième filetage (7; 7") évoluent en pente, en coupe axiale de la coque acétabulaire sur l'axe (3) de la coque acétabulaire, suivant un angle sensiblement de 70° à 90°, de préférence de 75° à 85°, et que également les flancs proximaux (6.2, 6.4) du premier filetage (2; 2") et les flancs proximaux (10.2, 10.4, 10.6, 10.8, 10.10) du deuxième filetage (7; 7") évoluent en pente, en coupe axiale de la coque acétabulaire, sur l'axe (3) de la coque acétabulaire, suivant un angle sensiblement de 70° à 90°, de préférence de 75° à 85°.

9. Coque acétabulaire selon la revendication 8, **caractérisée en ce que** les flancs distaux et proximaux (6.1, 6.3; 6.2, 6.4) du premier filetage (2; 2") évoluent en pente, en coupe axiale de la coque acétabulaire, sur l'axe (3) de la coque acétabulaire, suivant, en fonction de la grandeur, sensiblement le même angle, et que également les flancs distaux et proximaux (10.1, 10.3, 10.5, 10.7, 10.9; 10.2, 10.4, 10.6, 10.8, 10.10) du deuxième filetage (7; 7") évoluent en pente, en coupe axiale de la coque acétabulaire, sur l'axe (3) de la coque acétabulaire, suivant, sensiblement le même angle en fonction de la grandeur.

10. Coque acétabulaire selon la revendication 8 ou 9, **caractérisée en ce que** les flancs distaux (6.1, 6.3) du premier filetage (2) et les flancs distaux (10.1, 10.3, 10.5, 10.7, 10,9) du deuxième filetage (7) ainsi que les flancs proximaux (6.2, 6.4) du premier filetage (2) et les flancs proximaux (10.2, 10.4, 10.6, 10.8, 10.10) du deuxième filetage (7) évoluent sensiblement en parallèle les uns par rapport aux autres.

11. Coque acétabulaire selon l'une des revendications précédentes, **caractérisée en ce que** les pointes (4) du premier filetage (2; 2"), sur sensiblement toute la longueur du premier filetage (2; 2"), se trouvent sur une première surface (5; 5") sensiblement parallèle à la surface de gainage (1.1; 1.1") du corps de base (1; 1") et/ou les pointes (8) du deuxième filetage (7; 7"), sur sensiblement toute la longueur du deuxième filetage (7; 7"), se trouvent sur une deuxième surface (9; 9") sensiblement parallèle à la surface de gainage (1.1; 1.1") du corps de base (1; 1").

12. Coque acétabulaire selon la revendication 11, **caractérisée en ce que** la surface de gainage (1.1; 1.1") est sensiblement de forme hémisphérique.

13. Procédé pour la fabrication par usinage d'une coque acétabulaire filetée selon l'une des revendications précédentes, **caractérisé en ce que** la coque acétabulaire est usinée dans une première étape partielle pour produire le premier filetage (2; 2") avec au moins un premier outil (13; 13") et un deuxième outil (14; 14") et **en ce que** les flancs restants (10.3, 10.4, 10.5, 10.6) du deuxième filetage (7; 7") sont configurés pendant la première étape partielle ou dans une deuxième étape partielle.

14. Procédé selon la revendication 13, **caractérisé en ce que** le premier outil (13; 13") est configuré de telle sorte qu'il produit en même temps le rebord distal (6.1) du premier filetage (2; 2") et le rebord proximal (10.2) du segment distal contigu (7.1) du deuxième filetage (7; 7"), et le deuxième outil (14; 14") est configuré de telle sorte qu'il produit en même temps le rebord proximal (6.2) du premier filetage (2; 2") et le rebord distal (10.1) du segment proximal contigu (7.3) du deuxième filetage (7; 7").

15. Procédé selon la revendication 13 ou 14, **caractérisé en ce que** la coque acétabulaire est usinée pour la fabrication des flancs restants (10.3, 10.4, 10.5, 10.6) du deuxième filetage (7; 7") pendant la première étape partielle avec au moins un troisième outil (15; 15"), configuré de telle sorte qu'il produit en même temps le rebord proximal (10.4) d'un premier segment (7.2) du deuxième filetage (7; 7") et le rebord distal (10.3) du deuxième segment (7.1) du deuxième filetage (7; 7"), proximal contigu au premier segment (7.2).

16. Procédé selon la revendication 15, **caractérisé en ce que** la géométrie du troisième outil (15; 15") correspond à la géométrie du premier outil (13; 13") ou du deuxième outil (14; 14").

17. Procédé selon la revendication 13 ou 14, **caractérisé en ce que** la coque acétabulaire, pour la fabrication des flancs restants (10.3, 10.4, 10.5, 10.6) du deuxième filetage (7) pendant la deuxième étape partielle avec le premier outil (13) et/ou le deuxième outil (14), est usinée de telle sorte que sont produits en même temps, le rebord proximal (10.4) d'un premier segment (7.2) du deuxième filetage (7; 7") et le rebord distal (10.3) du deuxième segment (7.1) du deuxième filetage (7), proximal contigu au premier segment (7.2).

18. Procédé selon l'une des revendications 13 à 17, **caractérisé en ce que** le premier et le deuxième outils (13, 14; 13", 14") présentent sensiblement la même géométrie d'outil.

19. Processus selon l'une des revendications 13 à 18, **caractérisé en ce que**, lors de l'usinage de la coque acétabulaire, les outils (13, 14, 15; 13", 14", 15"), sont guidés sur une portion de trajectoire sensiblement parallèle à la surface de gainage par une translation parallèle (1.1; 1.1").

20. Procédé selon l'une des revendications 13 à 19, **caractérisé en ce que** le segment (13.3, 14.3, 15.3; 13.3", 14.3", 15.3") de l'outil (13, 14, 15; 13", 14", 15") chaque fois utilisé, générateur de la zone de transition (11) entre deux flancs (6.1, 10.2; 10.3, 10.4; 10.1, 6.2) dans le fond de filet (2.1; 2.1"), est configuré, en coupe axiale de la coque acétabulaire, sensiblement en forme d'arc, en particulier en forme d'arc de cercle.

21. Procédé selon la revendication 20, **caractérisé en ce que** le segment configuré en forme d'arc (13.3, 14.3, 15.3; 13.3", 14.3", 15.3") des outils (13, 14, 15; 13", 14", 15) est chaque fois guidé sensiblement tangentiellement à la surface de gainage (1.1; 1.1").

22. Procédé selon l'une des revendications 13 à 21, **caractérisé en ce que** des fraiseuses et/ou des outils de tournage configurés de manière adéquate sont utilisés comme outils (13, 14, 15; 13", 14", 15).
